Europäisches Patentamt

European Patent Office

Office européen des brevets

(11) Veröffentlichungsnummer : **0 094 513**
**B1**

(12) **EUROPÄISCHE PATENTSCHRIFT**

(45) Veröffentlichungstag der Patentschrift :
29.07.87

(21) Anmeldenummer : 83103763.5

(22) Anmeldetag : 19.04.83

(51) Int. Cl.⁴ : **A 61 K 9/26, A 01 N 25/10, C 05 G 3/00**

(54) Wirkstoff enthaltender Körper für die Langzeitabgabe.

(30) Priorität : 14.05.82 DE 3218150

(43) Veröffentlichungstag der Anmeldung :
23.11.83 Patentblatt 83/47

(45) Bekanntmachung des Hinweises auf die Patenterteilung : 29.07.87 Patentblatt 87/31

(84) Benannte Vertragsstaaten :
CH FR GB LI NL

(56) Entgegenhaltungen :
GB-A- 1 325 209
GB-A- 1 351 409
US-A- 3 887 699
US-A- 4 011 312
US-A- 4 293 539
Römpps Chemie Lexikon, Franckh'sche Verlagshandlung, Stuttgart, 7. Auflage, S. 2782-2783, "Poren"
Die Akte enthält technische Angaben, die nach dem Eingang der Anmeldung eingereicht wurden und die nicht in dieser Patentschrift enthalten sind.

(73) Patentinhaber : Akzo GmbH
Postfach 10 01 49 Kasinostrasse 19-23
D-5600 Wuppertal-1 (DE)

(72) Erfinder : Lange, Wolfgang, Dr. Dipl.-Chem.
Rosenstrasse 12
D-8753 Obernburg (DE)

**Beschreibung**

Die Erfindung betrifft Wirkstoffe wie Pharmazeutika, Pestizide, Peptide, Hormone, Enzyme u. dgl. enthaltende Körper, welche in der Lage sind, den Wirkstoff kontrolliert über lange Zeit an ihre Umgebung abzugeben.

Die Erfindung betrifft ferner ein Verfahren zur Herstellung derartiger Körper.

Man hat sich seit langem bemüht, Wirkstoffe, wie Arzneimittel, Hormone, Düngemittel, Schädlingsbekämpfungsmittel, Spurenelemente, Duftstoffe u. dgl. kontrolliert über längere Zeiträume abzugeben.

Bei der pharmazeutischen Anwendung werden die Wirkstoffe bis jetzt meistens oral als Tablette, Flüssigkeit, Dispersion oder als Injektion verabreicht, so daß das Medikament dann ziemlich schnell im Körper in einer hohen Konzentration vorliegt und die Konzentration im Laufe der Zeit abgebaut wird.

Da die Wirkstoffabnahme fast immer exponentiell erfolgt, ist leicht einzusehen, daß bei dieser Art von Wirkstoffgabe das therapeutisch sinnvolle Konzentrationsniveau nur kurzfristig eingestellt wird. Anfänglich kann ein zu hohes Niveau gegeben sein, was die Gefahr einer Überdosis mit sich bringt, später liegt dann über lange Zeit ein zu niedriges, ständig noch abnehmendes Niveau vor.

Für viele Anwendungsgebiete sind jedoch über einen längeren Zeitraum konstante Wirkstoffkonzentrationen erwünscht.

Analoge Verhältnisse sind auch auf Gebieten wie der Landwirtschaft und der Forstwirtschaft beispielsweise gegeben. Düngemittel z. B. werden meistens in Form eines Pulvers z. B. Ammoniumphosphat auf den Acker gebracht, wodurch zunächst eine hohe Konzentration des Düngemittels gegeben ist. Bei dem ersten Regen wird jedoch eine beachtliche Menge des Salzes weggeschwemmt bzw. versickert im Boden, so daß die Konzentration im Boden verarmt und der Pflanze wesentlich weniger des benötigten Wirkstoffs zur Verfügung steht.

Man hat sich bemüht, diese Nachteile zu beheben, indem man die Wirkstoffe in einem Depot unterbringt, z. B. in mikroporösen Pulvern, aus denen der Wirkstoff verlangsamt abgegeben werden soll.

Besonders in der Pharmazie haben sich sogenannte Mikrokapseln eingebürgert, bei denen der Wirkstoff von einer Membran umgeben ist, durch die der Wirkstoff durchdiffundieren kann. Von Nachteil bei den Mikrokapseln ist, daß die gewünschte Dickenverteilung der Wandstärke für eine erstrebte Abgaberate schwierig zu erreichen ist. Oft sind die Kapseln nicht ganz dicht, wenig beanspruchbar, was zu unkontrollierter Wirkstoffabgabe führen kann.

Man hat ferner versucht, den Wirkstoff in einer Matrix zu dispergieren, aus welcher der Wirkstoff durch Diffusion heraustreten kann. Nachteilig bei diesem Vorgehen ist, daß eine allmähliche Abnahme der Wirkstoffabgabe eintritt. Es ist also mit diesem Verfahren nicht möglich, über längere Zeit eine konstantbleibende Abgabe zu erreichen.

In der US-PS 3 887 699 und der US-PS 4 011 312 wird ein ähnliches Prinzip der Langzeitabgabe von Wirkstoffen beschrieben. Dort wird die Lehre vermittelt, einen Wirkstoff in einem polymeren Material zu dispergieren, das biskompatibel ist und aus dem der Wirkstoff an die Oberfläche diffundiert.

Von Nachteil bei diesen Verfahren ist, daß man in der Matrix nur bestimmte Höchstmengen des Wirkstoffs verteilen kann, da sonst die Handhabung des Materials schwierig wird und zudem der Wirkstoff leicht, bevor es zur Anwendung kommt, bereits aus dem Polymer herausschwitzen kann.

Es besteht deshalb noch ein Bedürfnis nach verbesserten Körpern, welche Wirkstoffe enthalten und diese auf kontrollierte Weise über lange Zeit hinaus an ihre Umgebung abgeben können.

Aufgabe der Erfindung ist es deshalb, ein Verfahren zur Verfügung zu stellen, mit dem sich solche Körper auf einfache und wirtschaftliche Weise herstellen lassen.

Aufgabe der Erfindung ist es ferner, wirkstoffenthaltende Polymere herzustellen, die als Matrix für den Wirkstoff dienen und selbst abbaubar sind.

Aufgabe der Erfindung ist es ferner, ein derartiges Wirkstoff/Polymersystem zur Verfügung zu stellen, in dem die Menge des eingelagerten Wirkstoffs in weiten Grenzen variiert werden kann, so daß die Intensität der konstanten Wirkstoffabgabe gut und reproduzierbar einstellbar ist.

Aufgabe der Erfindung ist es ferner, ein Polymer/Wirkstoffsystem zur Verfügung zu· stellen, das vielseitig einsatzfähig ist, auch in physiologischen Systemen, in denen biokompatible Polymere erforderlich sind.

Aufgabe der Erfindung ist es außerdem, ein System zur Verfügung zu stellen, das konstante Abgaberaten gewährleisten kann, d. h. nach einem Geschwindigkeitsgesetz nullter Ordnung, und das außerdem mit verschiedenen Wirkstoffen gleichzeitig ggf. jeweils in verschiedenen Konzentrationen beladen werden kann.

Diese Aufgabe wird durch ein Verfahren zur Herstellung eines wirkstoffenthaltenden Körpers für die Langzeitabgabe von Wirkstoffen gelöst, das dadurch gekennzeichnet ist, daß man ein pulver- oder granulatförmiges mikroporöses Polymer (A) mit Wirkstoff belädt und das den Wirkstoff enthaltende Polymer in einer Matrix aus abbaubarem Polymer (B) verteilt. Vorzugsweise wird die Verteilung des mikroporösen, Wirkstoff enthaltenden Polymers (A) in einer Schmelze eines abbaubaren Polymers (B) vorgenommen, dessen Schmelzpunkt unterhalb der Schmelztemperatur des mikroporösen Polymers (A) liegt. Anschließend wird die Schmelze abgekühlt. Als abbaubares Polymer (B) sind insbesondere Polymere geeignet, die im menschlichen oder tierischen Körper biologisch abbaubar sind. Hierzu sind vor allem die abbaubaren Copolymere der Milchsäure geeignet. Es ist zweckmäßig, wenn man als mikroporöses Polymer (A) ein ebenfalls abbaubares Polymer verwendet. Vielfach ist es günstig, wenn das mikroporöse Polymer (A) eine Abbaugeschwindigkeit aufweist, die niedriger ist als die Abbaugeschwindigkeit des Matrixpolymers (B).

In einer besonderen Ausführungsform wird als Polymer (B) ein weichgemachtes Polymer verwendet. Dazu können übliche Weichmacher eingesetzt werden, die selbstverständlich dem vorgesehenen Verwendungszweck des den Wirkstoff enthaltenden Körpers angemessen sein müssen. So dürfen für Anwendungen im menschlichen oder tierischen Körper keine giftigen oder schädlichen Weichmacher eingesetzt werden.

Bei Einsatz eines Weichmachers ist es auch gut möglich, anstelle eines von (A) unterschiedlichen Polymers (B) weichgemachtes Polymer (A) zu verwenden. Auch kann in vielen (B) selbst, wenn es Weichmacher hält, an sich einen höheren Schmelzpunkt als (A) aufweisen.

Die Verteilung des mit Wirkstoff beladenen Polymers (A) in Polymer (B) kann z. B. durch einfaches Einrühren in eine Schmelze von (B) oder durch Verkneten von (A) und (B) oder durch gemeinsames Extrudieren geschehen. Die Herstellung von Formkörpern ist sehr einfach.

Gegenstand der Erfindung ist ferner ein Wirkstoff enthaltender Körper für die Langzeitabgabe von Wirkstoffen, der gekennzeichnet ist durch eine Matrix aus abbaubarem Polymer (B), in der mit Wirkstoff beladene mikroporöse pulver- oder granulatförmige Polymere (A) gleichmäßig verteilt sind. Vorzugsweise handelt es sich bei dem Polymer (A) um ein ebenfalls abbaubares Polymer, dessen Abbaugeschwindigkeit geringer ist als die Abbaugeschwindigkeit des Matrixpolymeren (B). Vorzugsweise ist das abbaubare Polymere (B) ein Copolymer der Milchsäure.

Ein besonderer Vorteil der Erfindung ist, daß sie ermöglicht, sowohl feste als auch flüssige Wirkstoffe in bisher nicht erreichten Mengen in dem für die Wirkstoffabgabe bestimmten Körper zu speichern und über lange Zeit hinaus, die mehrere Monate bis zu einem halben Jahr und darüber hinaus dauern kann, kontrolliert abzugeben. Ein weiterer Vorteil der Erfindung ist, daß sie erlaubt, mit nur einem Körper eine Vielzahl von Wirkstoffen, die in ihren relativen Konzentrationen aufeinander abgestimmt sein können, kontrolliert abzugeben. So kann man üblicherweise verwendete Präparatekombinationen problemlos zusammenstellen, indem man jedes Pharmakon für sich, ggf. auch gemischt, jeweils separat in Pulver (A) einbringt, die so unterschiedlich beladenen Pulver (A) in der gewünschten Mengenkonstellation zusammenmischt und in der Matrix (B) verteilt.

Als Polymere im Rahmen der Erfindung kommen insbesondere infrage Polylactide auf der Basis von dl-Milchsäure, Polymere der l(+)-Milch-

säure, der d(—)-Milchsäure und der aus den hier erwähnten Milchsäuren herstellbaren Copolymeren.

Ferner ist es möglich eine oder mehrere der genannten Milchsäuren zusammen mit Cokomponenten zu polymerisieren. Als brauchbare Comonomere seien genannt Glycolid, Caprolactam, β-Propiolacton.

Die Erfindung wird durch folgendes Beispiel näher erläutert :

Beispiel 1

Zum Aufbau einer zylindrischen Releaseeinheit von 20 mm Länge und 2 mm Durchmesser mit einer geplanten Lebensdauer von ca. 6 Monaten wurde zunächst das Copolymer, bestehend aus 75 % (Gew) L(+)-Lactid und 25 % Glykolid, durch Blockpolymerisation mit 0,03 Gew.-% Zinnoctoat und 0,01 Gew.-% Laurylalkohol als Katalysator (Polymer 1979, Vol. 20, S. 1459) hergestellt.

Dazu wurden im Einzelnen 7,5 g L(+)-Dilactid zusammen mit 2,5 g Glykolid, (hergestellt nach Polymer 1979, Vol. 20, S. 1459) 2 h lang auf 200 °C im Bombenrohr erhitzt. Nach erfolgter Reaktion wurde der Inhalt abgekühlt, das Bombenrohr geöffnet und der Inhalt nach Aufschmelzen ausgegossen. Eine mit dem Copolymer durchgeführte Schmelzpunktbestimmung ergab einen Wert von 58 °C, der mit Literaturangaben (Polymer 1979, Vol. 20, S. 1463) übereinstimmt.

Als Träger für den Wirkstoff wurde zunächst mikroporöses Polylactid hergestellt. Zu diesem Zweck wurden 90,3 g d(—)-Polylactid mit einem Molekulargewicht von ca. 40 000 in 400 ml Xylol gelöst und anschließend ohne Rühren abkühlen gelassen. Die dabei erhaltene, homogen erscheinende Masse wurde abgenutscht und anschließend bei 60 °C vakuumgetrocknet. Hg-Intrusionsmessungen an diesem Pulver ergaben einen Wert von 55 % Porenvolumen.

Daraufhin wurden 10 g des mikroporösen Polylactid-Pulvers mittels Vakuum bei Zimmertemperatur mit 100 ml 50 %iger, äthanolischer Lösung von Cetyl-Alkohol als Testsubstanz beladen. Nach vorsichtigem Abnutschen der überschüssigen Flüssigkeit wurde das alkoholfeuchte Pulver bei Zimmertemperatur im Vakuum getrocknet. Gravimetrische Untersuchung des getrockneten Pulvers ergab einen Beladungsgrad des Pulvers von etwa 58 %.

Zur Herstellung des erfindungsgemäßen Relasekörpers wurden dann 10 g des Cetylalkoholbeladenen Polylactidpulvers zusammen mit 19 g des Copolymeren bei ca. 55 °C solange verknetet, bis die Gesamtmasse homogen erschien. Anschließend wurde das erhaltene Produkt mittels Laborextrusiometer bei 55 °C zu einer Nudel von 2 mm Durchmesser extrudiert.

Der erhaltene Formkörper hatte einen Beladungsgrad mit Cetyl-Alkohol von 20 %, bezogen auf das Gesamtgewicht.

## Patentansprüche

1. Verfahren zur Herstellung eines Wirkstoff enthaltenden Körpers für die Langzeitabgabe von Wirkstoffen, dadurch gekennzeichnet, daß man ein pulver- oder granulatförmiges mikroporöses Polymer (A) mit Wirkstoff belädt und das Wirkstoff enthaltende Polymer in einer Matrix aus abbaubarem Polymer (B) verteilt.

2. Verfahren nach Anspruch 1, dadurch gekennzeichnet, daß man das mikroporöse, Wirkstoff enthaltende Polymer (A) in einer Schmelze eines abbaubaren Polymers (B) verteilt, dessen Schmelzpunkt unterhalb der Schmelztemperatur des mikroporösen Polymers (A) liegt, und die Schmelze abkühlt.

3. Verfahren nach den Ansprüchen 1 bis 2, dadurch gekennzeichnet, daß man als abbaubares Polymer (B) im menschlichen oder tierischen Körper biologisch abbaubare Polymere verwendet.

4. Verfahren nach den Ansprüchen 1 bis 3, dadurch gekennzeichnet, daß man als abbaubares Polymer (B) Copolymere der Milchsäure verwendet.

5. Verfahren nach den Ansprüchen 1 bis 4, dadurch gekennzeichnet, daß man als mikroporöses Polymer (A) ein ebenfalls abbaubares Polymer verwendet.

6. Verfahren nach Anspruch 5, dadurch gekennzeichnet, daß man als mikroporöses Polymer (A) ein Polymer verwendet, dessen Abbaugeschwindigkeit niedriger ist als die Abbaugeschwindigkeit des Matrix-Polymers (B).

7. Verfahren nach den Ansprüchen 1 bis 6, dadurch gekennzeichnet, daß man als Polymer (B) ein weichgemachtes Polymer verwendet.

8. Verfahren nach Anspruch 7, dadurch gekennzeichnet, daß man als Polymer (B) weichgemachtes Polymer (A) verwendet.

9. Wirkstoff enthaltender Körper für die Langzeitabgabe von Wirkstoffen, gekennzeichnet durch eine Matrix aus abbaubaren Polymeren (B), in der mit Wirkstoff beladene mikroporöse pulver- oder granulatförmige Polymere (A) gleichmäßig verteilt sind.

10. Wirkstoff enthaltende Körper nach Anspruch 9, gekennzeichnet durch ein ebenfalls abbaubares Polymer (A), dessen Abbaugeschwindigkeit geringer ist als die Abbaugeschwindigkeit des Matrix-Polymeren (B).

11. Wirkstoff enthaltender Körper nach den Ansprüchen 9 bis 10, gekennzeichnet durch Copolymere der Milchsäure als abbaubares Polymer (B).

12. Wirkstoff enthaltender Körper nach den Ansprüchen 9 bis 11, dadurch gekennzeichnet, daß er verschiedene Wirkstoffe oder Wirkstoffkombinationen enthält.

## Claims

1. A process for the production of a body containing active substance for the sustained release of active substances, characterized in that a powder-form or granulate-form microporous polymer (A) is charged with active substance and the active-substance-containing polymer is dispersed in a matrix of degradable polymer (B).

2. A process as claimed in Claim 1, characterized in that the microporous, active-substance-containing polymer (A) is dispersed in a melt of a degradable polymer (B) of which the melting point is below the melting temperature of the microporous polymer (A) and the melt is cooled.

3. A process as claimed in Claims 1 and 2, characterized in that polymers which are biologically degradable in the human or animal body are used as the degradable polymer (B).

4. A process as claimed in Claims 1 to 3, characterized in that copolymers of lactic acid are used as the degradable polymer (B).

5. A process as claimed in Claims 1 to 4, characterized in that a degradable polymer is also used as the microporous polymer (A).

6. A process as claimed in Claim 5, characterized in that the microporous polymer (A) used is a polymer of which the degradation rate is lower than the degradation rate of the matrix polymer (B).

7. A process as claimed in Claims 1 to 6, characterized in that a plasticized polymer is used as the polymer (B).

8. A process as claimed in Claim 7, characterized in that plasticized polymer (A) is used as the polymer (B).

9. An active-substance-containing body for the sustained release of active substances, characterized by a matrix of degradable polymers (B) in which microporous powder-form or granulate-form polymers (A) charged with active substance are uniformly dispersed.

10. An active-substance-containing body as claimed in Claim 9, characterized by a similarly degradable polymer (A) of which the degradation rate is lower than the degradation rate of the matrix polymer (B).

11. An active-substance-containing body as claimed in Claims 9 and 10, characterized by copolymers of lactic acid as the degradable polymer (B).

12. An active-substance-containing body as claimed in Claims 9 to 11, characterized in that it contains various active substances or combinations of active substances.

## Revendications

1. Procédé de préparation d'un corps contenant une substance active et conçu pour la libération de substances actives sur une longue durée, caractérisé en ce que l'on charge de substance active un polymère (A) microporeux, se présentant sous forme de poudre ou de granulés, et que l'on disperse le polymère contenant la substance active dans une matrice constituée d'un polymère (B) dégradable.

2. Procédé conforme à la revendication 1, caractérisé en ce que l'on disperse le polymère (A) microporeux, contenant la substance active, dans une masse fondue d'un polymère (B) dégradable, dont le point de fusion est inférieur à la température de fusion du polymère (A) microporeux, et l'on refroidit la masse fondue.

3. Procédé conforme aux revendications 1 à 2, caractérisé en ce que l'on utilise, en tant que polymère dégradable (B), des polymères dégradables biologiquement dans les corps humains ou animaux.

4. Procédé conforme aux revendications 1 à 3, caractérisé en ce que l'on utilise, en tant que polymère dégradable (B), des copolymères de l'acide lactique.

5. Procédé conforme aux revendications 1 à 4, caractérisé en ce que l'on utilise, en tant que polymère microporeux (A), un polymère également dégradable.

6. Procédé conforme à la revendication 5, caractérisé en ce que l'on utilise, en tant que polymère microporeux (A), un polymère dont la vitesse de dégradation est plus faible que la vitesse de dégradation du polymère (B) constituant la matrice.

7. Procédé conforme aux revendications 1 à 6, caractérisé en ce que l'on utilise, en tant que polymère (B), un polymère plastifié.

8. Procédé conforme à la revendication 7, caractérisé en ce que l'on utilise, en tant que polymère (B), le polymère (A) plastifié.

9. Corps contenant une substance active et conçu pour la libération de substances actives sur une longue durée, caractérisé par une matrice en polymère dégradable (B), dans laquelle sont répartis les polymères (A) microporeux, chargés de substance active, et se présentant sous forme de poudre ou de granulés.

10. Corps contenant une substance active conforme à la revendication 9, caractérisé par un polymère (A) également dégradable, dont la vitesse de dégradation est plus faible que la vitesse de dégradation du polymère (B) constituant la matrice.

11. Corps contenant une substance active conforme aux revendications 9 à 10, caractérisé par des copolymères de l'acide lactique utilisés à titre de polymère dégradable (B).

12. Corps contenant une substance active conforme aux revendications 9 à 11, caractérisé en ce qu'il contient différentes substances actives ou combinaisons de substances actives.